# EUROPÄISCHE PATENTSCHRIFT

(11) **EP 0 407 434 B1**
(45) Veröffentlichungstag und Bekanntmachung des Hinweises auf die Patenterteilung: **22.09.1993**
(21) Anmeldenummer: 89904030.7
(22) Anmeldetag: 30.03.1989
(51) Int. Cl.: A61B 17/58, F16B 23/00, F16B 35/04, F16B 37/14

(54) **KNOCHENSCHRAUBE**
BONE SCREW
VIS POUR OS

(30) Priorität: 02.04.1988 DE 3811345
(43) Veröffentlichungstag der Anmeldung: 16.01.1991
(73) Patentinhaber: AESCULAP AG, D-78501 Tuttlingen (DE)
(72) Erfinder: MÜHLING, Joachim, D-6967 Buchen (DE); LUTZE, Theodor, D-7201 Balgheim (DE); PISTNER, Hans, D-8700 Würzburg (DE)
(74) Vertreter: Hoeger, Stellrecht & Partner
(86) Internationale Anmeldenummer: EP8900344
(87) Internationale Veröffentlichungsnummer: WO8909030

(56) Entgegenhaltungen:
- EP-A- 0 213 479
- EP-A- 0 260 222
- DE-A- 2 036 512
- DE-A- 2 945 886
- DE-A- 3 434 807
- DE-A- 3 513 997
- DE-U- 8 804 456
- FR-A- 1 477 637
- GB-A- 637 683
- GB-A- 2 084 468
- US-A- 4 013 071

## Beschreibung

Die Erfindung betrifft eine Knochenschraube aus resorbierbarem Kunststoffmaterial, mit einem mit einem Außengewinde versehenen Schaft, in dem sich ein nach oben offener, konzentrisch zum Schaft angeordneter und sich im wesentlichen über die Schaftlänge erstreckender Einsteckkanal für ein Eindrehwerkzeug befindet, der einen von der Kreisform abweichenden und dem Querschnitt des Eindrehwerkzeuges entsprechenden Querschnitt aufweist.

Bisher sind Knochenschrauben zum Verschrauben von Implantaten mit Knochensubstanz oder zum Verschrauben benachbarter Knochenfragmente überwiegend aus rostfreiem Stahl oder Titan hergestellt worden. Diese Konstruktionen weisen aufgrund der mechanischen Eigenschaften dieses Materials eine hohe Festigkeit auf, die es erlaubt, die Konstruktion an Schrauben für die Holz- und Blechbearbeitung anzulehnen. Nachteilig ist bei derartigen Schrauben jedoch, daß sie nach Abschluß des Heilungsprozesses wieder aus dem Körper entfernt werden müssen, daß also eine weitere Operation notwendig wird.

Es sind in neuerer Zeit Knochenschrauben aus Kunststoffmaterial, insbesondere aus resorbierbarem Kunststoffmaterial bekanntgeworden, beispielsweise aus Polylactid oder Poly-L-Lactid. Diese haben den großen Vorteil, daß sie nach Abschluß des Heilungsprozesses vom Körper resorbiert werden, so daß eine zusätzliche Operation zur Entfernung derartiger Implantate überflüssig wird. Als nachteilig hat sich jedoch bei derartigen Kunststoffschrauben herausgestellt, daß das Kunststoffmaterial eine wesentlich geringere Festigkeit aufweist als konventionelle Stahl- oder Titanschrauben. Es besteht daher die Gefahr, daß beim Eindrehen derartiger Knochenschrauben in vorgebohrte und vorgeschnittene Gewindelöcher durch die vom Schraubendreher auf die Kunststoffschraube ausgeübte Torsionsspannung die Kunststoffschraube abgedreht und damit zerstört wird. Man kann dem zwar in gewissem Umfange dadurch begegnen, daß man veränderte Kerndurchmesser, Flankenwinkel und Gewindesteigungen verwendet, jedoch gelingt es trotz dieser Maßnahmen nicht in allen Fällen, beim Einschrauben dieser Schrauben mittels Innensechskant-, Schlitz- oder Kreuzschlitzschraubendreher, die in herkömmlicher Weise am Kopf der Knochenschraube angreifen, eine Zerstörung der Schrauben zu vermeiden.

Die Gefahr des Abscherens einer solchen Knochenschraube aus resorbierbarem Material wird bei einer bekannten Knochenschraube dadurch verhindert, daß diese einen sich über die gesamte Schaftlänge der Schraube erstreckenden Einsteckkanal für ein unrundes Eindrehwerkzeug aufweist, das sich somit formschlüssig über die gesamte Schaftlänge an die Wand des Einsteckkanals anlegt. Dadurch wird das Drehmoment über die gesamte Schaftlänge verteilt in die Schraube eingeleitet (EP-A-0260222). Nachteilig ist aber, daß sich dadurch eine Schwächung der Festigkeit der Knochenschraube nach dem Einsetzen in den Knochen ergibt. Die Resorption des Kunststoffmaterials geht von der Oberfläche aus und führt daher bei einer solchen Knochenschraube rasch dazu, daß das hochmolekulare Kunststoffmaterial abgebaut wird.

Der Erfindung liegt die Aufgabe zugrunde, eine derartige Knochenschraube trotz eines durchgehenden Einschraubkanals im Schaft so auszubilden, daß die Festigkeit der Knochenschraube möglichst lange erhalten bleibt, auch wenn die Resorption des Schraubenmaterials bereits einsetzt.

Diese Aufgabe wird bei einer Knochenschraube der eingangs beschriebenen Art erfindungsgemäß dadurch gelöst, daß in den Einsteckkanal ein diesen vollständig ausfüllender Verschlußstift aus resorbierbarem Material eingesetzt wird, dessen Querschnitt so bemessen ist, daß er die Schraube nicht aufweitet.

Der Verschlußstift verhindert nicht nur das unerwünschte Eindringen von Körperflüssigkeit und anderen Körpersubstanzen in den Einsteckkanal, sondern er erhöht auch die Festigkeit der Knochenschraube, so daß eine durch die Ausbildung eines Einsteckkanals möglicherweise eintretende Schwächung der Knochenschraube weitgehend wieder aufgehoben wird.

Der Einsteckkanal kann am Einschraubende der Knochenschraube verschlossen sein. Dadurch erfährt das Eindrehwerkzeug eine Festlegung seiner Eintauchtiefe, das heißt in diesem Falle ist die Position des Eindrehwerkzeuges im Einsteckkanal genau definiert.

Bei einer anderen Ausführungsform kann der Einsteckkanal die Knochenschraube vollständig durchsetzen. Dies hat den Vorteil, daß der Schaft auf seiner gesamten Länge eine entsprechende Verstärkung und eine über die gesamte Schaftlänge gleichmäßige Einschraubkrafteinleitung erfährt.

Bei einem bevorzugten Ausführungsbeispiel ist vorgesehen, daß der Querschnitt des Einsteckkanales ein gleichseitiges Dreieck ist. Bei dieser Ausführung wird der Querschnitt des Schaftes besonders wenig geschwächt. Es ist jedoch auch möglich, Querschnitte in Form eines Rechtecks, insbesondere eines Quadrats, eines regelmäßigen Vieleckes oder eines Sterns zu verwenden.

Es kann weiterhin vorgesehen sein, daß der Schaft an einem Ende eine kopfförmige Verbreiterung aufweist und daß der Einsteckkanal durch die Verbreiterung hindurchläuft. Eine solche kopfförmige Verbreiterung dient als Anschlag für die Einschraubtiefe der Knochenschraube.

Bei einer ersten Ausführungsform ist der Verschlußstift im Einsteckkanal im Preßsitz fixierbar, wobei sich vorzugsweise der Einsteckkanal oder der Verschlußstift in Einschubrichtung im Querschnitt verjüngen beziehungsweise erweitern. Beim Einsetzen des Verschlußstiftes wird dieser sich also klemmend an die Innenwand des Einsteckkanales anlegen und dadurch dauerhaft im Einsteckkanal fixiert.

Bei einer anderen Ausgestaltung ist vorgesehen, daß der Verschlußstift im Einsteckkanal mit der Knochenschraube verklebt oder verschweißt ist.

Bei einer anderen Ausgestaltung ist der Verschlußstift im Einsteckkanal durch eine elastische Rastverbindung im Formschluß fixierbar. In allen Fällen kann der Verschlußstift eine seine Einschubtiefe begrenzende Verbreiterung tragen, wobei eine Rastverbindung vorzugsweise im Bereich der Verbreiterung des Verschlußstiftes angeordnet ist.

Günstig ist es auch, wenn der Verschlußstift oder die Knochenschraube im Bereich der Rastverbindung in axialer Richtung geschlitzt sind. Dadurch wird die Einsteckbewegung des Verschlußstiftes erleichtert, da die aneinander anliegenden Teile der Knochenschraube und des Verschlußstiftes aufgrund der Schlitze elastisch gegeneinander verbiegbar sind.

Die nachfolgende Beschreibung bevorzugter Ausführungsformen der Erfindung dient im Zusammenhang mit der Zeichnung der näheren Erläuterung. Es zeigen:
- Figur 1:: eine teilweise aufgebrochen dargestellte Knochenschraube mit eingesetztem Eindrehwerkzeug ;
- Figur 2:: eine Draufsicht auf ein erstes bevorzugtes Ausführungsbeispiel einer Knochenschraube mit einem Einsteckkanal mit quadratischem Querschnitt;
- Figur 3:: eine Ansicht ähnlich Figur 2 mit einem Einsteckkanal mit dreieckigem Querschnitt;
- Figur 4:: eine Ansicht ähnlich Figur 2 mit einem Einsteckkanal mit sechseckigem Querschnitt;
- Figur 5:: eine Ansicht ähnlich Figur 2 mit einem Einsteckkanal mit sternförmigem Querschnitt;
- Figur 6:: eine Knochenschraube mit einem dazupassenden Verschlußstift;
- Figur 7:: eine Ansicht ähnlich Figur 6 mit einem am unteren Ende geschlitzten Verschlußstift mit Rastnasen;
- Figur 8:: eine Ansicht der Knochenschraube der Figur 7 mit eingesetztem und verrastetem Verschlußstift;
- Figur 9:: eine Ansicht ähnlich Figur 7 mit einem abgeänderten, verrastbaren Verschlußstift;
- Figur 10:: eine Ansicht der Knochenschraube der Figur 9 mit eingerastetem Verschlußstift;
- Figur 11:: eine Ansicht des oberen Teils einer im Kopfbereich geschnittenen Knochenschraube und des oberen Teils eines Verschlußstiftes und
- Figur 12:: eine Ansicht des oberen Teils der Knochenschraube der Figur 11 mit eingesetztem Verschlußstift.

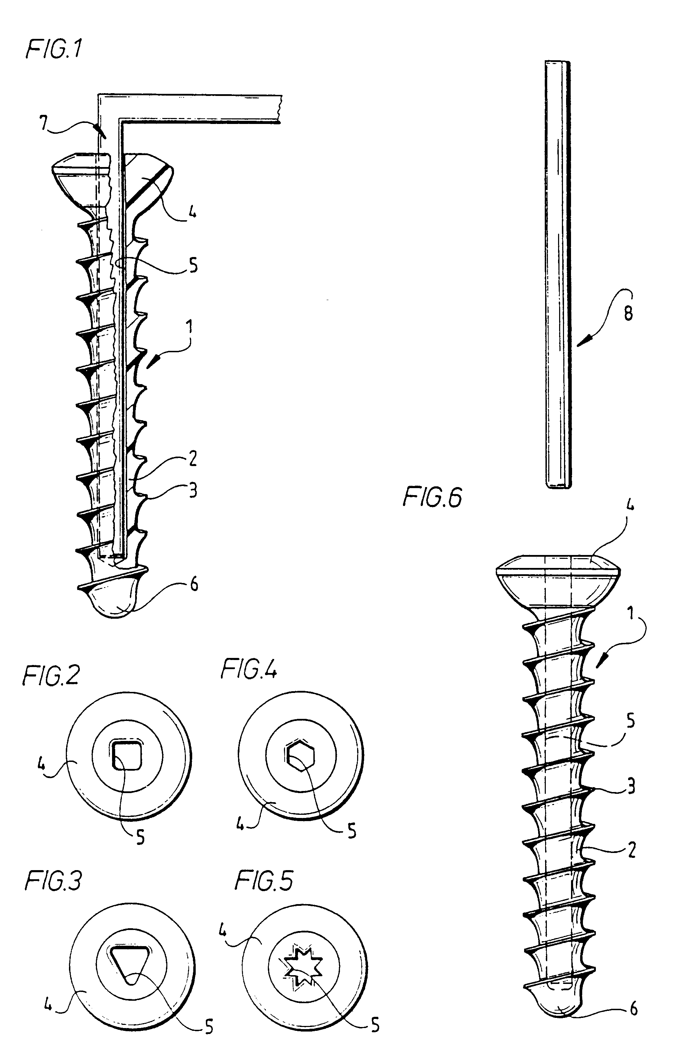

Die in Figur 1 dargestellte Knochenschraube 1 weist einen Schaft 2 mit Gewindegängen 3 und einem verbreiterten Kopf 4 auf. Durch den Kopf 4 hindurch erstreckt sich ein zentraler Einsteckkanal 5 in das Innere des Schaftes 2 hinein und verläuft dort bis fast an das einschraubseitige Ende 6 der Knochenschraube. Der Einsteckkanal 5 weist über seine gesamte Länge einen gleichbleibenden, von der Kreisform abweichenden Querschnitt auf, vorzugsweise hat der Querschnitt die Form eines gleichseitigen Dreiecks, wie dies in Figur 3 dargestellt ist. Bei anderen Ausführungsbeispielen kann der Querschnitt die Form eines Rechteckes, insbesondere eines Quadrates (Figur 2), die Form eines regelmäßigen Vieleckes, beispielsweise eines Seckseckes(Figur 4) oder die Form eines Sternes (Figur 5) haben.

In den an der Kopfseite offenen Einsteckkanal 5 kann ein Eindrehwerkzeug 7 eingesetzt werden, das üblicherweise komplementär zum Querschnitt des Einsteckkanales 5 ausgebildet ist und dadurch eine formschlüssige Verbindung mit den Seitenwänden des Einsteckkanales 5 herstellt. Das Eindrehwerkzeug 7 wird über die gesamte Länge des Einsteckkanales 5 eingeführt und erstreckt sich somit im wesentlichen über die gesamte Länge des Schaftes 2. Beim Drehen des Eindrehwerkzeuges 7 um die Längsachse der Knochenschraube 1 ergibt sich eine kraftschlüssige Drehverbindung zwischen Eindrehwerkzeug und Knochenschraube, wobei die Krafteinleitung über die gesamte Länge des Einsteckkanales gleichmäßig erfolgt, das heißt die Eindrehkraft wird über die gesamte Länge verteilt. Da als Eindrehwerkzeug 7 ein Material hoher Festigkeit verwendet werden kann, beispielsweise Stahl, gelingt es auf diese Weise auch bei relativ geringer Scherfestigkeit des Knochenschraubenmaterials, große Torsionskräfte auf die Schraube auszuüben, ohne die Zerstörung der Knochenschraube zu riskieren. Insbesondere können die Drehkräfte auch in den Bereich optimal eingeführt werden, in dem gerade die größten Torsionskräfte auftreten, weil in diesem Bereich Gewindegänge in Knochenmaterial eingeschraubt werden. Zusätzlich verstärkt der als stabiler Kern wirkende Teil des Eindrehwerkzeuges die Gesamtfestigkeit der Knochenschraube während des Eindrehvorganges.

Nach dem Eindrehen kann das Eindrehwerkzeug 7 in einfacher Weise aus dem Einsteckkanal wieder herausgezogen werden.

Bei dem in Figur 1 dargestellten Ausführungsbeispiel ist der Einsteckkanal 5 am einschraubseitigen Ende 6 verschlossen. Es wäre jedoch auch möglich, den Einsteckkanal 5 durchgehend auszubilden.

Zum Verschließen des sacklochartigen Einsteckkanales 5 ist ein Verschlußstift 8 vorgesehen, der nach dem Eindrehen der Knochenschraube mittels des Eindrehwerkzeugs 7 in den Einsteckkanal 5 eingesteckt werden kann und diesen nach außen verschließt. In dem in Figur 6 dargestellten einfachsten Fall ist der Verschlußstift 8 zylindrisch ausgebildet und entspricht in seiner Länge der Länge des Einsteckkanales 5. Der Querschnitt des Verschlußstiftes 8 entspricht dem Querschnitt des Einsteckkanales 5, so daß der Verschlußstift 8 den Einsteckkanal 5 vollständig ausfüllt. Ebenso wie die Knochenschraube aus resorbierbarem Material besteht, ist auch der Verschlußstift 8 aus resorbierbarem Material hergestellt, wobei insbesondere günstig ist, wenn Knochenschraube und Verschlußstift aus dem gleichen Material bestehen. Dieses Material kann außerdem durch eingebettete Fasern verstärkt sein.

Um den Verschlußstift 8 in dem Einsteckkanal 5 zu fixieren, kann dieser so dimensioniert sein, daß er im Klemm- oder Reibsitz im Einsteckkanal gehalten wird. Zu diesem Zweck ist es auch möglich, den Querschnitt des Einsteckkanales in Einsteckrichtung zu reduzieren oder den Verschlußstift 8 im Querschnitt an seinem hinteren Ende zu vergrößern, so daß eine Klemmung erst erfolgt, wenn der Verschlußstift schon weitgehend in den Einsteckkanal 5 eingeschoben ist.

Anstelle einer Klemm- oder Reibsitzfixierung kann der Verschlußstift auch in die Knochenschraube eingeklebt sein, zum Beispiel mit Hilfe eines gewebeverträglichen Fibrinklebers.

Eine andere Möglichkeit der dauerhaften Fixierung des Verschlußstiftes im Einsteckkanal 5 ergibt sich durch eine Verschweißung. Erwärmt man die Knochenschraube und den Verschlußstift im Kopfbereich der Knochenschraube, so kann man eine Verschweißung der beiden Teile in diesem Bereich erreichen.

Eine spezielle Form der Verschweißung ist dann einsetzbar, wenn der Verschlußstift rotationssymmetrisch ausgebildet ist und in dem Einsteckkanal verdrehbar ist. Der Einsteckkanal hat zwar immer eine nicht rotationssymmetrische Form, damit das Eindrehwerkzeug 7 drehfest eingesetzt werden kann, man kann aber trotzdem in einen dreieckigen, sternförmigen oder quadratischen Einsteckkanal einen Verschlußstift mit einem kreisförmigen Querschnitt einsetzen, so daß dieser Verschlußstift linienförmig an der Wand des Einsteckkanales anliegt. Durch eine rasche Drehung des Verschlußstiftes um dessen Längsachse ergibt sich im Anlagebereich zwischen Einsteckkanal-Wand und Verschlußstift aufgrund der Reibung eine Erwärmung, die bis zum Aufschmelzen führen kann, so daß der Verschlußstift mit dem Wandmaterial verschweißt. Ein solches Reib-Schweiß-Verfahren kann dazu dienen, den Verschlußstift in dem Einsteckkanal dauerhaft festzulegen.

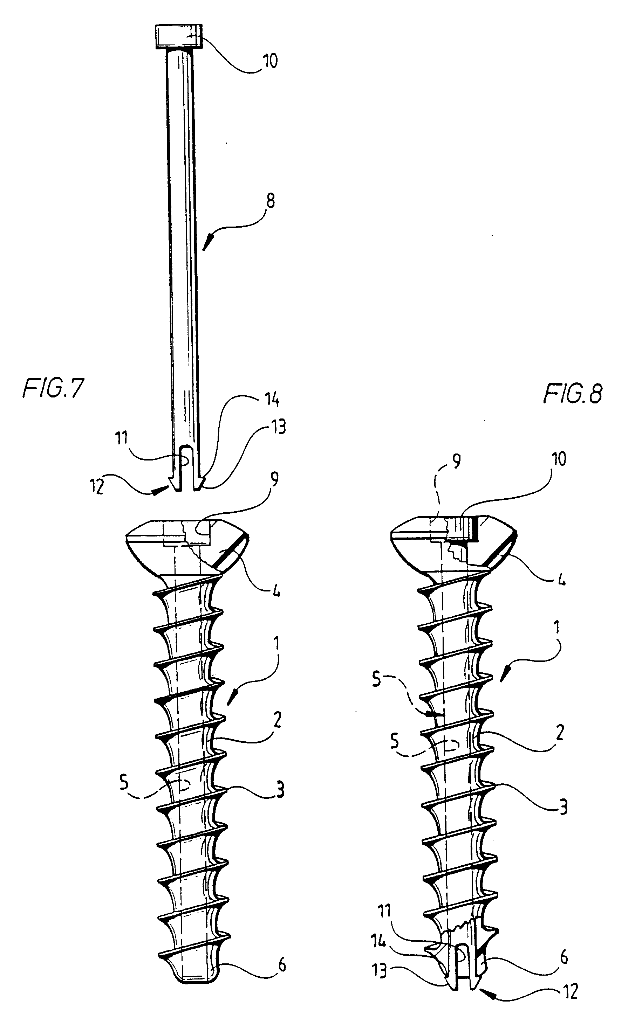

Bei dem in den Figuren 7 und 8 dargestellten Ausführungsbeispiel weist die Knochenschraube 1 einen durchgehenden Einsteckkanal 5 auf, der im Bereich des Kopfes 4 der Knochenschraube 1 in einer stufenförmig erweiterten Ausnehmung 9 endet.

Der in diesen Einsteckkanal 5 einführbare Verschlußstift 8 weist eine zu der Ausnehmung 9 komplementäre, kopfförmige Verbreiterung 10 auf, außerdem trägt der Verschlußstift 8 am gegenüberliegenden Ende einen in Axialrichtung verlaufenden Längsschlitz 11, der die anliegenden Wandbereiche voneinander trennt. Am Ende des Verschlußstiftes 8 sind nach außen abstehende Rastnasen 12 gehalten, die in Einschubrichtung abgeschrägte Aufgleitflächen 13 aufweisen, während an der Rückseite rechtwinklige Rastflächen 14 angeordnet sind. Beim Einschieben des Verschlußstiftes 8 in den Einsteckkanal 5 werden die Rastnasen 12 elastisch in Richtung auf die Längsachse des Einsteckkanales verbogen, wobei diese elastische Verbiegung durch den Längsschlitz 11 möglich wird. Sobald der Verschlußstift 8 vollständig eingeschoben ist, schnappen die Rastnasen 12 nach außen und liegen mit ihren radial abstehenden Rastflächen 14 an der Spitze der Knochenschraube 1 an, so daß durch diese Rastnasen 12 einerseits und die in die Ausnehmung 9 eintretende Verbreiterung 10 andererseits der Verschlußstift 8 in der Knochenschraube in axialer Richtung unverschieblich gehalten ist.

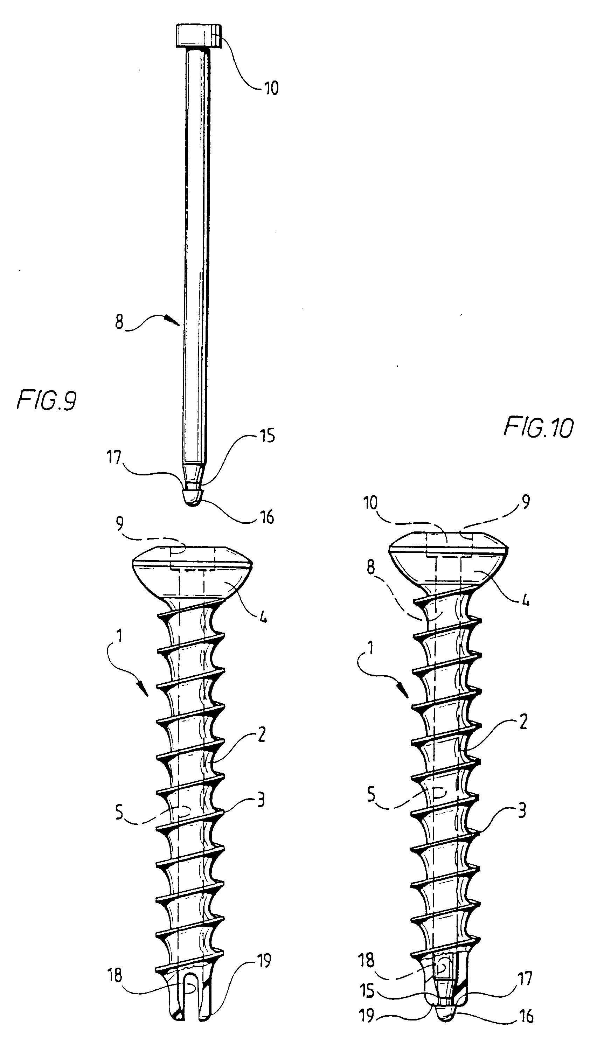

Das in den Figuren 9 und 10 dargestellte Ausführungsbeispiel unterscheidet sich von dem der Figuren 7 und 8 im wesentlichen nur dadurch, daß in diesem Falle der Verschlußstift 8 keinen Längsschlitz und keine nach außen abstehenden Rastnasen trägt, sondern im Gegenteil eine gegenüber dem Außendurchmesser zurückgesetzte Rastnut 15 mit schrägen Aufgleitflächen 16 und einer radial nach außen abstehenden Rastfläche 17. Dafür ist die Knochenschraube im Bereich ihres vorderen Endes mit einem Längsschlitz 18 und nach innen gerichteten Rastvorsprüngen 19 versehen. Diese Rastvorsprünge 19 greifen bei voll eingeschobenem Verschlußstift 8 in die Rastnut 15 ein und legen sich so an die Rastfläche 17, daß ein Herausbewegen des Verschlußstiftes 8 aus dem Einsteckkanal 5 verhindert wird. In diesem Falle ermöglicht der Längsschlitz 18 ein elastisches Auseinanderbiegen der Rastvorsprünge 19, das heißt der Verschlußstift 8 bildet zusammen mit der Knochenschraube 1 eine elastische, unlösbare Rastverbindung aus.

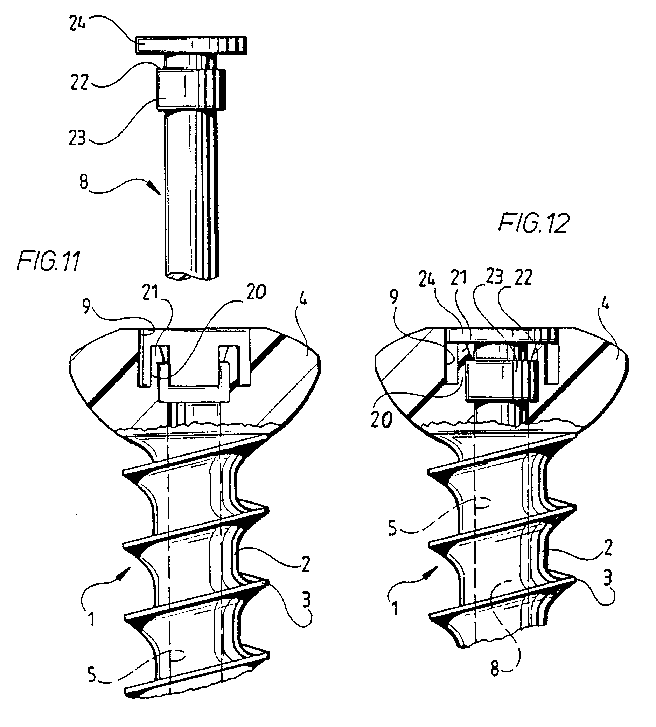

Bei dem in den Figuren 11 und 12 dargestellten Ausführungsbeispiel ragen in die Ausnehmung 9 im Kopf 4 der Knochenschraube 1 senkrecht nach oben stehende Rastarme 20, die an ihrem oberen Ende nach innen vorspringende Rastnasen 21 aufweisen. Diese Rastnasen 21 hintergreifen eine radial nach außen abstehende Rastfläche 22 an einer Verdickung 23 des Verschlußstiftes 8 und fixieren dadurch den Verschlußstift gegen ein Herausschieben aus dem Einsteckkanal. Gegen ein zu weites Einschieben ist der Verschlußstift 8 dabei durch eine tellerartige Verbreiterung 24 gesichert, die sich bei eingeschobenem Verschlußstift 8 an den Rastarmen 20 abstützt und dabei die Ausnehmung 9 abschließt (Figur 12).

## Patentansprüche

1. Knochenschraube aus resorbierbarem Kunststoffmaterial, mit einem mit einem Außengewinde versehenen Schaft, in dem sich ein nach oben offener, konzentrisch zum Schaft (2) angeordneter und sich im wesentlichen über die Schaftlänge erstreckender Einsteckkanal (5) für ein Eindrehwerkzeug (7) befindet, der einen von der Kreisform abweichenden und dem Querschnitt des Eindrehwerkzeuges (7) entsprechenden Querschnitt aufweist,
gekennzeichnet durch einen in den Einsteckkanal (5) einsteckbaren, diesen vollständig ausfüllenden Verschlußstift (8) aus resorbierbarem Material, dessen Querschnitt so bemessen ist, daß er die Schraube nicht aufweitet.

2. Knochenschraube nach Anspruch 1, dadurch gekennzeichnet, daß der Einsteckkanal (5) am Einschraubende (6) der Knochenschraube (1) verschlossen ist.

3. Knochenschraube nach Anspruch 1, dadurch gekennzeichnet, daß der Einsteckkanal (5) die Knochenschraube (1) vollständig durchsetzt.

4. Knochenschraube nach einem der voranstehenden Ansprüche, dadurch gekennzeichnet, daß der Querschnitt des Einsteckkanales (5) ein gleichseitiges Dreieck ist.

5. Knochenschraube nach einem der Ansprüche 1 bis 3, dadurch gekennzeichnet, daß der Querschnitt des Einsteckkanales (5) ein Rechteck und vorzugsweise ein Quadrat ist.

6. Knochenschraube nach einem der Ansprüche 1 bis 3, dadurch gekennzeichnet, daß der Querschnitt des Einsteckkanales (5) ein regelmäßiges Vieleck ist.

7. Knochenschraube nach einem der Ansprüche 1 bis 3, dadurch gekennzeichnet, daß der Querschnitt des Einsteckkanales (5) sternförmig ist.

8. Knochenschraube nach einem der voranstehenden Ansprüche, dadurch gekennzeichnet, daß der Schaft (2) an einem Ende eine kopfförmige Verbreiterung (4) aufweist und der Einsteckkanal (5) durch die Verbreiterung (4) hindurchläuft.

9. Knochenschraube nach einem der voranstehenden Ansprüche, dadurch gekennzeichnet, daß der Verschlußstift (8) im Einsteckkanal (5) im Preßsitz fixierbar ist.

10. Knochenschraube nach Anspruch 9, dadurch gekennzeichnet, daß sich der Einsteckkanal (5) oder der Verschlußstift (8) in Einschubrichtung im Querschnitt verjüngen beziehungsweise erweitern.

11. Knochenschraube nach einem der Ansprüche 1 oder 8, dadurch gekennzeichnet, daß der Verschlußstift (8) im Einsteckkanal (5) mit der Knochenschraube (1) verklebt oder verschweißt ist.

12. Knochenschraube nach einem der Ansprüche 1 bis 8, dadurch gekennzeichnet, daß der Verschlußstift (8) im Einsteckkanal (5) durch eine elastische Rastverbindung (12, 14; 17, 19; 21, 22) durch Formschluß fixierbar ist.

13. Knochenschraube nach einem der voranstehenden Ansprüche, dadurch gekennzeichnet, daß der Verschlußstift (8) eine seine Einschubtiefe begrenzende Verbreiterung (10; 24) trägt.

14. Knochenschraube nach Anspruch 12 oder 13, dadurch gekennzeichnet, daß die Rastverbindung (21, 22) im Bereich der Verbreiterung (24) des Verschlußstiftes (8) angeordnet ist.

15. Knochenschraube nach einem der Ansprüche 12 bis 14, dadurch gekennzeichnet, daß der Verschlußstift (8) oder die Knochenschraube (1) im Bereich der Rastverbindung (12, 14; 17, 19) in axialer Richtung geschlitzt sind.

## Claims

1. Bone screw made of resorbing plastic material, comprising a shaft provided with an external thread, an insertion channel (5) for a tightening tool (7) being located in said shaft, said insertion channel being open at the top, arranged concentrically to the shaft (2) and extending essentially along the length of the shaft, the cross section of said insertion channel being non-circular and corresponding to the cross section of the tightening tool (7), characterized by a closure pin (8) made of resorbing material insertable into and completely filling the insertion channel (5), the cross section of said pin being dimensioned such that it does not expand the screw.

2. Bone screw as defined in claim 1, characterized in that the insertion channel (5) is closed at the screw-in end (6) of the bone screw (1).

3. Bone screw as defined in claim 1, characterized in that the insertion channel (5) penetrates the bone screw (1) completely.

4. Bone screw as defined in one of the preceding claims, characterized in that the cross section of the insertion channel (5) is an equilateral triangle.

5. Bone screw as defined in any of claims 1 to 3, characterized in that the cross section of the insertion channel (5) is a rectangle and preferably a square.

6. Bone screw as defined in any of claims 1 to 3, characterized in that the cross section of the insertion channel (5) is a regular polygon.

7. Bone screw as defined in any of claims 1 to 3, characterized in that the cross section of the insertion channel (5) is star-shaped.

8. Bone screw as defined in any of the preceding claims, characterized in that the shaft (2) has a head-shaped widened portion (4) at one end and the insertion channel (5) extends through the widened portion (4).

9. Bone screw as defined in any of the preceding claims, characterized in that the closure pin (8) is adapted to be fixed in position in the insertion channel (59 in a press fit.

10. Bone screw as defined in claim 9, characterized in that the insertion channel (5) or the closure pin (8) taper or widen in cross section in the direction of insertion.

11. Bone screw as defined in any of claims 1 to 8, characterized in that the closure pin (8) is bonded or welded to the bone screw (1) in the insertion channel (5).

12. Bone screw as defined in any of claims 1 to 8, characterized in that the closure pin (8) is adapted to be fixed in position in the insertion channel in a positively connected manner by an elastic locking connection (12, 14; 17, 19; 21, 22).

13. Bone screw as defined in any of the preceding claims, characterized in that the closure pin (8) bears a widened portion (10; 24) limiting its insertion depth.

14. Bone screw as defined in claim 12 or 13, characterized in that the locking connection (21, 22) is arranged in the region of the widened portion (24) of the closure pin (8).

15. Bone screw as defined in any of claims 12 or 14, characterized in that the closure pin (8) of the bone screw (1) are slotted in axial direction in the region of the locking connection (12, 14; 17, 19).

## Revendications

1. Vis pour os en matière plastique résorbable, comprenant une tige munie d'un filetage mâle dans laquelle se trouve un canal d'insertion (5), pour un outil de vissage (7), qui est ouvert vers le haut, concentrique à la tige (2), qui s'étend sensiblement sur la longueur de la tige et qui présente une section droite qui n'est pas de forme circulaire et qui correspond à la section droite de l'outil de vissage (7), caractérisée par une tige d'obturation (8) en matière résorbable qui peut être insérée dans le canal d'insertion (5), qui le remplit totalement et dont la section droite est choisie de telle manière qu'il n'élargit pas la vis.

2. Vis pour os selon la revendication 1, caractérisée en ce que le canal d'insertion (5) est fermé à l'extrémité insérée (6) de la vis pour os (1).

3. Vis pour os selon la revendication 1, caractérisée en ce que le canal d'insertion (5) traverse totalement la vis pour os (1).

4. Vis pour os selon l'une des revendications précédentes, caractérisée en ce que la section droite du canal d'insertion (5) est un triangle équilatéral.

5. Vis pour os selon l'une des revendications 1 à 3, caractérisée en ce que la section droite du canal d'insertion (5) est un rectangle et de préférence un carré.

6. Vis pour os selon l'une des revendications 1 à 3, caractérisée en ce que la section droite du canal d'insertion (5) est un polygone régulier.

7. Vis pour os selon l'une des revendications 1 à 3, caractérisée en ce que la section droite du canal d'insertion (5) est en forme d'étoile.

8. Vis pour os selon l'une des revendications précédentes, caractérisée en ce que la tige (2) comporte à une extrémité un élargissement (4) en forme de tête et le canal d'insertion (5) s'étend à travers l'élargissement (4).

9. Vis pour os selon l'une des revendications précédentes, caractérisée en ce que la tige d'obturation (8) peut être fixée dans le canal d'insertion (5) par ajustage serré.

10. Vis pour os selon la revendication 9, caractérisée en ce que la section droite du canal d'insertion (5) ou de la tige d'obturation (8) se rétrécit ou s'élargit dans la direction d'introduction.

11. Vis pour os selon l'une des revendications 1 ou 8, caractérisée en ce que la tige d'obturation (8) est collée ou soudée à la vis pour os (1) dans le canal d'insertion (5).

12. Vis pour os selon l'une des revendications 1 à 8, caractérisée en ce que la tige d'obturation (8) peut être fixée dans le canal d'insertion (5) par assemblage de forme à l'aide d'une liaison à crans élastique (12, 14 ; 17, 19 ; 21, 22).

13. Vis pour os selon l'une des revendications précédentes, caractérisée en ce que la tige d'obturation (8) porte un élargissement (10 ; 24) qui limite sa profondeur d'introduction.

14. Vis pour os selon la revendication 12 ou 13, caractérisée en ce que la liaison à crans (21, 22) est agencée dans le domaine de l'élargissement (24) de la tige d'obturation (8).

15. Vis pour os selon l'une des revendications 12 à 14, caractérisée en ce que la tige d'obturation (8) ou la vis pour os (1) est fendue en direction axiale dans le domaine de la liaison à crans (12, 14 ; 17, 19).
